(19) **Europäisches Patentamt**
**European Patent Office**
**Office européen des brevets**

(11) **EP 2 016 390 B1**

(12) **EUROPEAN PATENT SPECIFICATION**

(45) Date of publication and mention
of the grant of the patent:
**10.04.2013 Bulletin 2013/15**

(21) Application number: **07748071.3**

(22) Date of filing: **27.04.2007**

(51) Int Cl.:
*G01N 21/31* (2006.01)  *G01N 33/487* (2006.01)
*G01N 33/49* (2006.01)  *G01N 21/27* (2006.01)

(86) International application number:
**PCT/SE2007/000406**

(87) International publication number:
**WO 2007/129948 (15.11.2007 Gazette 2007/46)**

(54) **A method and a system for quantitative hemoglobin determination**

Verfahren und Vorrichtung zur quantitativen Hämoglobinbestimmung

Procédé et système de détermination quantitative d'hémoglobine

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **05.05.2006 SE 0601025**

(43) Date of publication of application:
**21.01.2009 Bulletin 2009/04**

(73) Proprietor: **HemoCue AB
262 23 Ängelholm (SE)**

(72) Inventor: **PETTERSSON, Joakim
SE-260 83 Vejbystrand (SE)**

(74) Representative: **Ahlberg, Lena Camilla et al
Awapatent AB
Box 1066
251 10 Helsingborg (SE)**

(56) References cited:
EP-A2- 1 069 427     WO-A1-03/056327
WO-A1-2006/040387     US-A- 5 898 487
US-A- 6 084 661     US-A1- 2005 036 147
US-B2- 6 831 733

- ENEJDER A.M.K. ET AL.: 'Influence of cell shape and aggregate formation on the optical properties of flowing whole blood' APPLIED OPTICS, OPT. SOC. AMERICA vol. 42, no. 7, 01 March 2003, pages 1384 - 1394, XP003016213
- GRECO F.A.: 'Reflectance spectroscopy of clotting blood: A description of the time-dependent behavior' ANNUAL MEETING OF THE COLLEGE OF AMERICAN PATHOLOGISTS; SAN DIEGO, CA, USA; ARCHIVES OF PATHOLOGY & LABORATORY MEDICINE vol. 128, no. 2, 10 September 2003 - 14 September 2003, pages 173 - 180, XP003016214

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention concerns an analysis method and a system for performing this analysis. Specifically the invention concerns a method for determination of hemoglobin in unaltered whole blood and a system which can be used in this determination.

Background Art

A disposable cuvette for sampling a fluid, mixing the sample with a reagent and directly making optical analyses of the sample mixed with the reagent is previously known from U.S. Patent No. 4,088,448. This known cuvette has several advantages as it i.a. simplifies the sampling procedure, reduces the number of utensils and considerably improves the accuracy of analysis by making the analysing procedure independent of the operating technique of the operator making the analysis. A cuvette construction based on the same principle and with improved flow characteristics is disclosed in the U.S. Patent No. 5,674,457.

A disposable cuvette developed according to these patents is currently widely used for hemoglobin measurement (Hb determination) of undiluted whole blood. To this end the cuvette cavity has been pre-treated with a reagent, such that when a blood sample is drawn into the cuvette, the walls of the red blood cells are disintegrated and a chemical reaction is initiated. The result of the reaction allows Hb determination by absorption measurement directly through the transparent walls of the cuvette which, in the measuring zone, also called the optical window, has a predetermined and accurately defined distance between the inner surfaces of the opposing planar walls. The measurement method is based on a modified azidmethemoglobin method according to Vanzetti, G., "An azide-methaemoglobin method for haemoglobin determination in blood", Am.J. Lab.& Clin. Med. 67, 116-126 (1966).

The spectrophotometric measurements are made at 570 and 880 nm. This quantitative measurement method based on dry chemistry has met with considerable success as can be seen in e.g. the article by von Schenck, H., Falkensson, M. and Lundberg, B., "Evaluation of 'HemoCue', a new device for determining hemoglobin", Clinical Chemistry, vol 32, No 3, pages 526-529, 1986, as the method gives equal or even superior results in comparison with the results obtained with standardised wet methods for the determination of Hb. The reagent used is comprised of sodium deoxycholate which hemolyses the red blood cells, sodium azide and sodium nitrite, which converts hemoglobin to azidmethemoglobin.

Due to the hygroscopic properties of the reagents used, the shelf life is limited and the storage of the cuvettes in sealed packages including a drying agent is required. Even more troublesome is the fact that, in climates with high humidity, the cuvette has to be used within a few minutes after the removal from the package, as otherwise the reagents will be destroyed and the measurement will be inaccurate and thus useless.

The problems originating from the hygroscopic properties of the reagents used may however be eliminated as it has been found that these reagents must not be used, as disclosed in U.S. Patent No. 6,638,769, according to which the first absorption measurement is performed at a wavelength range 490-520 nm directly on the sample in the microcuvette. According to the invention disclosed in this patent application it is however necessary that the blood is hemolysed before the measurement is performed. The cuvette cavity must thus include a hemolysing agent for disintegrating the red blood cells and releasing the hemoglobin contained in these cells. The necessity of using a hemolysing agent when performing photometric absorbance measurements of hemoglobin in a blood sample is also disclosed in e.g. the U.S. Patent No. 5,064,282 (Artel).

Quantitative methods for optical determination of hemoglobin in whole blood without using hemolysing agent are known but these methods have in common that they are all comparatively complicated. This depends above all on the inhomogeneity of the blood due to the high concentration of red blood cells, a consequence of which is that light is scattered upon interaction with these particles of inhomogeneous blood samples. Accordingly the light is not transmitted directly through the sample but deflected over a range of scattering angles. Another factor that causes problems is the fact that blood may contain as many as five different species of hemoglobin. Patent publications addressing these problems are i.a. the U.S. Patent No. 6,262,798 (Shepherd) and WO 01/53806 (Radiometer).

According to the invention disclosed in the U.S. Patent No. 6,262,798 a plurality of wavelengths are needed in order to achieve a correct measurement. The fact that many wavelengths are needed makes the spectrophotometer comparatively complicated. The wavelengths are selected by their ability to distinguish the hemoglobin species at minimum scatter and maximum absorbance. The patent also discloses the use of a large detector which reduces the problem of scattering beyond the detection range.

WO 01/53806 discloses an apparatus which is especially applicable for optical measurements on whole blood. This apparatus comprises an absorption filter or an interference filter, which provides correction for variations in the detector sensitivity and in the effective optical path length as observed upon varying level of scattering. The apparatus uses a large detector for detecting scattered light transmitted through the absorption filter or the interference filter.

US 5,898,487 discloses an apparatus for determining the concentrations of n hemoglobin derivatives in a sample of undiluted, unhemolyzed whole blood provided in a cuvette, employs a device producing a primary light beam formed of at least n substantially monochromatic, narrow-band components of different wavelengths. At least two measuring geometries are provided for spectral detection of a collimated central beam and at least one scatter beam, and detection of the central beam departing from a first measuring location in the cuvette takes place in a first measuring position on the axis of the primary beam, and detection of the scatter beam takes place in a second measuring position if it departs from the first measuring location in the cuvette, or in the first measuring position if it departs from a second measuring location in the cuvette.

US 6,084,661 discloses a compact microspectro-photometer for measuring HbCO in a sample of whole undiluted blood, preferably from a finger-prick and provided in a capillary tube, which comprises subjecting the blood sample to radiation at three wavelengths. A photodetector determines absorption of the blood at each wavelength. A processor uses determination at one of the wavelengths to select one of several calibration curves relating to blood hematocrit of the patient. Using the selected curve, the CO level is determined. The wavelengths are preferably 548 nm, 810 nm, and 950 nm. The absorption at each wavelength is determined substantially simultaneously.

US 2005/036147 discloses a method for determination of an analyte concentration in a sample which includes the analyte and a substance. The method includes providing absorption data, by using infrared (IR) spectroscopy, of the sample. The method further includes providing reference absorption data of the substance and includes calculating a substance contribution of the absorption data. The method further includes subtracting the substance contribution from the absorption data, thereby providing corrected absorption data substantially free of a contribution from the substance.

In U.S. Patent No. 6,831,733, it has been shown that an accurate determination of the total amount of hemoglobin in whole blood can be made not only without using a hemolysing agent but also without using a plurality of wavelengths as disclosed in the U.S. Patent No. 6,262,798. According to U.S. Patent No. 6,831,733, the total amount of hemoglobin in whole blood could be determined by performing two absorbance measurements, at a first wavelength in the range 490-520 nm, and at a second wavelength at which the absorption is substantially smaller than at the first wavelength. The concentration of hemoglobin in the sample may then be determined by processing results of the first and second absorption measurements. The difference in absorption between the first and second absorption measurements is mainly due to the difference in absorption of the hemoglobin. However, there are other factors that affect the difference in absorption. The most important other factor is the difference in scattering in the sample. According to U.S. Patent No. 6,831,733, the effect of scattering is regarded as being dependent of the absorbance measured in the second absorption measurement. Thus, it has been unexpectedly found that the concentration of hemoglobin in the sample could be measured as the difference in absorption between merely two absorption measurements, by using a term to compensate for scattering. The compensation term is dependent on the result of the second absorption measurement.

Summary of the Invention

It is an object of the present invention to provide a rapid, quantitative method for the determination of hemoglobin in unaltered whole blood.

A second object is to provide a method for the determination of hemoglobin in unaltered whole blood, which may be performed in a microcuvette that may also be used for acquiring a sample of blood.

A third object is to provide a simple method of processing results of absorption measurements for determination of hemoglobin in unaltered whole blood.

A fourth object is to provide a system for implementing the methods for the determination of hemoglobin in unaltered whole blood.

Other objects will be apparent from the following description and the accompanying claims.

In accordance with claim 1 a method for providing such a hemoglobin determination is provided.

In accordance with claim 7 a system for providing such a hemoglobin determination is provided.

According to the invention it has been unexpectedly found that quantitative determinations of hemoglobin can not only be easily performed directly on an unaltered, i.e. undiluted and unhemolyzed, sample of whole blood, but may also be achieved by simply conducting two absorption measurements at different wavelengths and processing these results.

The determination may be performed on a blood sample without using hygroscopic reagents, such as sodium azide and sodium nitrate, or a hemolysing agent. Thus, the hemoglobin determination is based on measurements of absorption, while the hemoglobin is bound inside red blood cells. The hemoglobin level may thus be determined without lysing the red blood cells to release the hemoglobin.

Further, it has now been unexpectedly realized that it is not even necessary to compensate for the effect of scattering of the red blood cells. A difference between two absorption measurement reflects both an effect due to absorption of light in hemoglobin and an effect due to scattering of light by the red blood cells. However, by using a delay in performing the absorption measurements, it may be controlled at which time the absorption measurements are performed. In development of the invention it has been observed that the results of the absorption measurements vary with time in respect to the acquiring of a blood sample. The result of the first absorption measurement varies more heavily. This implies that the difference between the two absorption measurements will vary depending on at which point of time the measurements were performed. Thus, the delay in performing the absorption measurements may control at which time the absorption measurements are performed and the processing of the results may be calibrated accordingly to output a correct value of the hemoglobin concentration in the blood sample.

In particular, the difference between the absorption measurement results decrease heavily the first period of time after the blood sample is acquired. Thereafter, the difference is relatively stable for a period of time and after a while the difference start to increase again. The delay may thus be adapted to allow the measurements to be performed at a time when the measurement results are relatively stable. This makes the result of the determined hemoglobin concentration quite insensitive to the exact point of time at which it is performed. Thus, the result is not dependent on whether the acquired sample is presented instantly to the measurement apparatus or a minute passes before the acquired sample is presented to the measurement apparatus.

It is believed that the variation in measurement results is at least partly due to movements within the sample as the sample is acquired into the cuvette. After a while, the movements have been allowed to settle and the measurement results are more stable. The effect of scattering of the red blood cells is then also diminished and, therefore, the processing of the results of the absorption measurements need not account for effects of the scattering of the red blood cells being different for the first and second absorption measurements. The measurement results may later start to increase again as the red blood cells are beginning to settle within the bottom portion of the cuvette. The red blood cells will therefore collect at the bottom of the cuvette, which will increase the effect of scattering.

Thus, according to the invention, the hemoglobin determination is performed in a simple manner. Only two absorption measurements are needed using a sample of unaltered whole blood. Further, the hemoglobin content may be determined using a simple algorithm for processing the results of the two absorption measurements. This implies that it is easy to calibrate an instrument to present correct results with the algorithm. However, the ease of calibration is achieved at the cost of a somewhat prolonged time for obtaining an analysis result, since the analysis need to be delayed in order to ensure that effects of scattering need not be accounted for.

In the context of this application, the term "absorption measurement" should be construed as a measurement related to the absorption in a sample. In an absorption measurement, the intensity of light detected after interacting with a sample is compared with the intensity of light irradiated on the sample. The detected light corresponds to the transmittance through the sample. The light that does not reach the detector is considered to be absorbed. Thus, in the results of the measurements the transmittance may be used instead of the absorption. As the transmittance is the inverse of the absorption, detecting transmittance would still be an absorption measurement. However, the measured absorption does not only correspond to light that has been truly absorbed in the sample, since some of the light has been scattered in the sample so that it does not reach the detector.

Further, the term "determination" should be construed as the measurement not necessarily obtaining an absolutely exact value of the concentration of hemoglobin in the sample. Thus, the concentration of hemoglobin is "determined" within reasonable margins of error such that the result not merely gives an order of magnitude of the concentration, while not necessarily giving an absolute value.

The processing may be performed by a predetermined algorithm. This implies that the algorithm may be programmed into an instrument and that the instrument may directly return analysis results after the absorption measurements have been performed.

The processing may determine the concentration of hemoglobin in the sample by computing the following formula:

$$[\text{Tot Hb}] = (Abs_1 - Abs_2) \cdot k_1 + k_2$$

wherein [Tot Hb] is the total concentration of hemoglobin in the sample, $Abs_1$ is the measured absorbance of the first absorption measurement, $Abs_2$ is the measured absorbance of the second absorption measurement, and $k_1$ and $k_2$ are calibration coefficients, which depend on the measurement arrangement.

This implies that the total concentration of hemoglobin in a sample is simply determined by computing a difference between the two absorption measurements. There is only a need for two calibration coefficients which may handle the slope of the curve and the offset of the curve from the origin of coordinates. Thus, it is only necessary to determine two values of calibration coefficients, whereby calibration of instruments may be achieved in a simple manner.

The presenting may comprise placing the cuvette in a holder of an instrument for performing absorption measurements. This implies that the cuvette may be guided to a correct position for absorption measurements within the instrument.

The instrument is arranged to enable absorption measurements giving the sample a possibility to settle appropriately such that the scattering effects of the red blood cells will not affect the analysis result.

The delaying is made by monitoring results of absorption measurements and, when the results are substantially constant, allowing the first and second absorption measurements to be performed for determining the concentration of hemoglobin in the sample. This implies that a first check is made to ensure that the absorption measurements are performed at a point of time where the effect of scattering in the blood sample will not significantly affect the determining of the concentration of hemoglobin. When this has been established, the absorption measurements are performed. Alternatively, the first check may be made in any other way to determine that the movements within the blood sample have settled.

The first absorption measurement may be performed at a wavelength in the range 500 - 510 nm, more preferably at 506 nm. In the wavelength range of 490 - 520 nm, and especially 500 - 510 nm, the absorptions of the five different forms of hemoglobin, namely oxy-, deoxy-, carboxy-, met- and sulfhemoglobin, are significant and similar. Thus, the absorption in this wavelength range will depend only slightly on the distribution between the different forms of hemoglobin in the blood. Especially, at 506 nm, the difference between the absorbances of oxy- and deoxyhemoglobin is close to zero. Since these forms of hemoglobin are predominant in normal blood, the absorption of oxy- and deoxyhemoglobin could advantageously be used for determining an absorption coefficient for relating a measured absorption to the concentration of hemoglobin at 506 nm. Accordingly, some assumptions are made regarding the contents of different forms of hemoglobin in the blood sample. Thus, the hemoglobin determination will not be as accurate or the processing of the measurement results will have to be modified, if a measurement is made on a blood sample having a very differing distribution of the forms of hemoglobin. Further, the measurements will only determine the total concentration of hemoglobin and not the concentrations of the specific forms of hemoglobin.

The second absorption measurement may be performed at a wavelength in the range 650 - 1200 nm, more preferably in the range 850 - 910 nm, most preferably in the range 860 - 900 nm. At these wavelength ranges, the absorption of hemoglobin is significantly lower than at the first wavelength. Further, the wavelength should be chosen such that absorption of other substances in the blood sample is substantially the same at the first and second wavelengths. This implies that the difference in absorption may be related to the concentration of hemoglobin in the sample. The second wavelength may advantageously be selected in the range 860 - 900 nm, whereby the absorption of other substances will not affect the analysis result.

The cuvette may have an optical path length of less than 1 mm, more preferably less than 0.2 mm. This ensures that a sufficient intensity of light may be transmitted through the sample in order for the absorption measurements to be statistically significant. A smaller optical path would allow higher intensities of light to be detected.

The cuvette may have an optical path length in the range 0.05 - 0.2 mm. This implies that light is transmitted through a sufficient amount of blood in order to enable determination of hemoglobin concentration, while sufficient intensities of light may be detected without use of a strong light source.

The detector may have a detecting area of a size such that essentially only directly transmitted light is detected. This implies that light that is scattered into a substantially different direction is not detected, whereby the absorption measurement determines the amount of light being transmitted without being scattered or absorbed. Thus, the measurement may assume that all light that is scattered into another direction is not detected.

The detector may be arranged closer than 10 mm to the sample holder. This further implies that only light being scattered in small angles is detected.

The means for emitting light may comprise one light source, which is arranged to emit light at the first wavelength and to emit light at the second wavelength. Then, filters may be used for ensuring that the sample is illuminated with the correct wavelength. Alternatively, the means for emitting light may comprise a first light source, which is arranged to emit light at the first wavelength, and a second light source, which is arranged to emit light at the second wavelength. The different light sources may then be appropriately turned on and off in order to illuminate the sample with the correct wavelength.

Brief Description of Drawings

The invention will now by way of example be described in more detail with reference to the accompanying drawings.

Fig. 1 is a flow chart of a method according to the invention.
Fig. 2 is a schematic diagram of the absorbance of hemoglobin.
Fig. 3 is a diagram illustrating how results of an algorithm for determining the hemoglobin concentration vary over time after obtaining a sample.

Fig. 4 is a schematic view of a system according to the invention.

Detailed Description of a Presently Preferred Embodiment

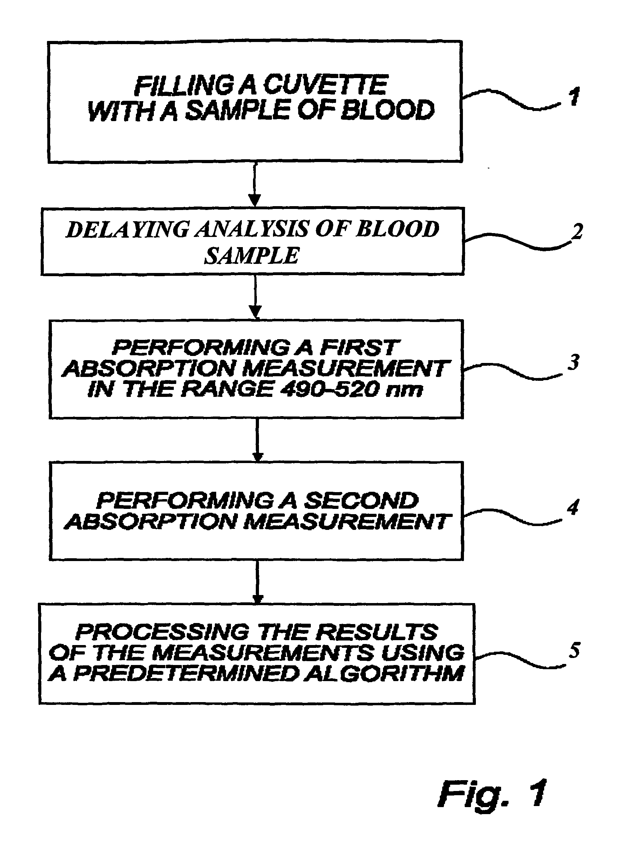

Referring now to Fig. 1, a method for hemoglobin determination according to the invention will be described. First, a disposable, capillary cuvette is filled with a sample of unaltered whole blood, step 1. Thus, a sample which is to be analysed is obtained. The analysis of the blood sample is delayed, step 2. This may be achieved by means of an instrument for performing analysis being arranged to start analysis at a predetermined period of time after placement of the cuvette in a holder of the instrument. This delay will allow movements within the sample to settle, whereby effects of red blood cells scattering light will be diminished. Then, a first absorption measurement on the sample is performed at a wavelength in the range 490 - 520 nm, step 3. Further, a second absorption measurement is performed on the sample, step 4. The second absorption measurement is performed at a wavelength in the range 650 - 1200 nm. This second absorption measurement is chosen such that the difference in absorbance between the two absorption measurements can be related only to the hemoglobin concentration in the sample, as will be described in further detail below. Finally, the results of the measurements are processed, step 5, using a predetermined algorithm for determining the concentration of hemoglobin in the sample.

The disposable microcuvette used according to the present invention may be of the type disclosed in the US patent 4,088,448 or preferably in the US patent 5,674,457. The cuvette may be defined as a unitary body member including at least one cavity with an optical window (measuring zone) wherein two, plane or curved, surfaces facing the cavity are placed at a predetermined distance from one another and thus define a predetermined optical path length. This distance between the surfaces defining the measuring zone is a critical parameter in providing the proper optical path length for the hemoglobin measurement. The optical path length should be less than 1 mm in order to ensure that the intensity of light transmitted through a sample in the cuvette is sufficient to enable determination of hemoglobin in the sample. In a preferred embodiment, this distance is less than 0.2 mm, and more preferably between 0.05 and 0.2 mm. The distance between the inner surfaces of the rest of the cavity is preferably in the order of 0.1-2 mm which is effective to permit the sample to enter the cavity by capillary force through the cavity inlet, which is communicating with the exterior of the body member. Furthermore, the cavity has a predetermined fixed volume of less than about 25 μl. No active additives, such as reagents or hemolysing agents, are necessary for the determination according to the inventive method.

The cuvettes according to the present invention may be formed by any suitable material, which allows the formation of the necessary tight tolerance levels. Preferably the cuvette is manufactured by injection moulding of a transparent polymeric material.

In order to overcome problems related to the capillary filling of the cuvette it may be necessary to pretreat the inner surfaces of the cuvette in order to impart a hydrophilic character to these surfaces. This may be achieved by coating the surfaces with a suitable detergent, such as Brij 35. Another possibility is to select a hydrophilic material for the manufacturing of the cuvette.

A feature of the inventive method is that the absorption determination should be carried out at a wavelength in a range of 490 - 520 nm, more preferably in the range 500-510 nm, and most preferably at 506 nm. The secondary absorption measurement is preferably performed at a wavelength in the range 650 - 1200 nm, more preferably in the range 850 - 910 nm, and most preferably in the range 860 - 900 nm.

The absorption measurements are performed directly on the whole blood in the sample, i.e. the blood is unaltered (undiluted and unhemolyzed).

In the wavelength range of 490 - 520 nm, the absorptions of the five different forms of hemoglobin, namely oxy-, deoxy-, carboxy-, met- and sulfhemoglobin, are similar and significant. Thus, the absorption in this wavelength range will depend only slightly on the distribution between the different forms of hemoglobin in the blood. Especially, at 506 nm, the difference between the absorbances of oxy- and deoxyhemoglobin is close to zero. Since these forms of hemoglobin are predominant in normal blood, the absorption of oxy- and deoxyhemoglobin could advantageously be used for determining an absorption coefficient for relating a measured absorption to the concentration of hemoglobin at 506 nm. Accordingly, some assumptions are made regarding the contents of different forms of hemoglobin in the blood sample. Thus, the hemoglobin determination will not be as accurate or the processing of the measurement results will have to be modified, if a measurement is made on a blood sample having a very differing distribution of the forms of hemoglobin. Further, the measurements will only determine the total concentration of hemoglobin and not the concentrations of the specific forms of hemoglobin.

A second absorption measurement is performed at a wavelength, where the absorption of light in blood is substantially smaller. Such an absorption measurement could suitably be performed at a wavelength in the range 650 - 1200 nm. The differences between the absorption measurements is then considered to be due to absorption of hemoglobin.

However, the scattering of light varies with the concentration of hemoglobin in the sample, but the scattering of light is not only dependent on the concentration of hemoglobin. The scattering of light is due to light interaction with particles in the blood, such as red blood cells, white blood cells, and lipid particles. According to the invention, it has been found that by delaying the analysis, movements within the sample will settle and the effect of scattering in the sample will decrease. Thus, it has been unexpectedly found that calibration of a measurement instrument may be used to handle the scattering effects and that the concentration of hemoglobin in a sample may be directly related to the difference in absorption between the two absorption measurements.

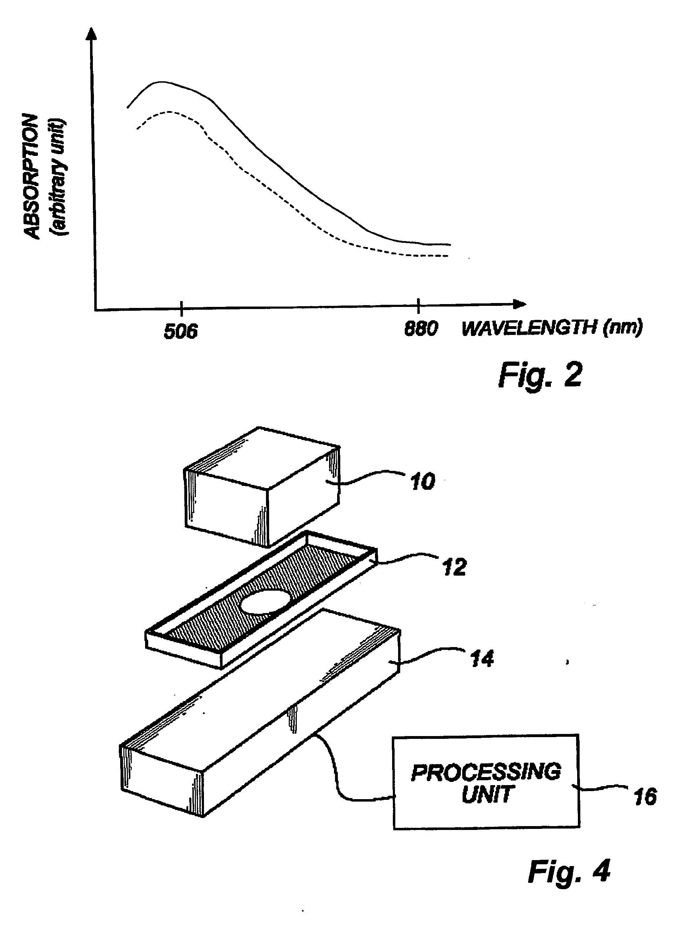

The principle of an algorithm for determining the concentration of hemoglobin will now be described with reference to the schematic diagram in Fig. 2. In Fig. 2, the solid line schematically illustrates measured absorption in a first sample having a high concentration of hemoglobin. The absorption includes both true absorption and light scattered so that it does not reach a detector. The dashed line in Fig. 2 schematically illustrates measured absorption in a second sample having a lower concentration of hemoglobin. The measured absorptions are performed after the sample has been allowed to settle. It should be noted that the schematic diagram in Fig. 2 only emphasizes the main features of absorption of samples of whole blood, and does not illustrate absorption of real samples. As can be seen in Fig. 2, for both samples there is a substantial difference in absorption between a first wavelength at 506 nm and a second wavelength at 880 nm. As described above, this difference depends on the concentration of hemoglobin in the sample and the amount of scattering of light in the sample. When movements of cells within the sample have been allowed to settle, the effect of scattering of light is reduced. Thus, the difference in absorption is then mainly dependent on the concentration of hemoglobin in the sample. It has now been found that acceptable results of determining the concentration of hemoglobin in a sample may be obtained by simply using the difference in absorption between the two absorption measurements and relating it to the concentration of hemoglobin by calibration constants.

According to the above, the results of the absorption measurements should be processed for determining the concentration of hemoglobin in the sample. This processing may be performed by a predetermined algorithm. This algorithm calculates the concentration of hemoglobin according to the above-described scheme.

The processing may determine the concentration of hemoglobin in the sample by computing the following formula:

$$\left[\text{Tot Hb}\right] = \left(Abs_1 - Abs_2\right) \cdot k_1 + k_2$$

wherein [Tot Hb] is the total concentration of hemoglobin in the sample, $Abs_1$ is the measured absorbance of the first absorption measurement, $Abs_2$ is the measured absorbance of the second absorption measurement, and $k_1$ and $k_2$ are calibration coefficients, which depend on the measurement arrangement. The calibration coefficients $k_1$ and $k_2$ may be specific for each instrument used for hemoglobin determination.

The calibration coefficients may be determined by performing absorption measurements on a set of blood samples having known concentrations of hemoglobin. These calibration measurements may be performed when an instrument is manufactured. Further, calibration measurements may be performed at regular intervals in order to ensure that the instrument returns correct analysis results. Then, the calibration coefficients may be updated regularly to handle any differences in the performance of the instrument.

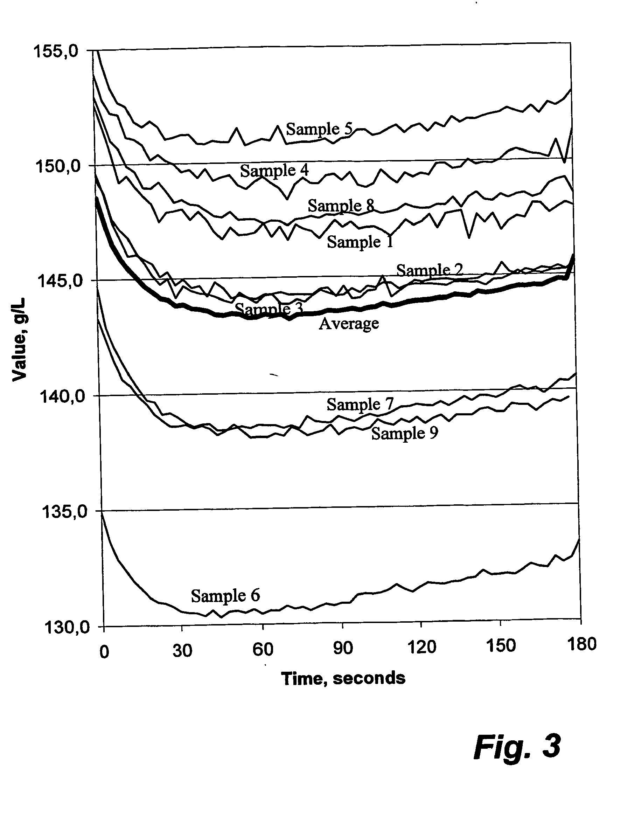

In Fig. 3, the time dependence of performing analysis is shown. Fig. 3 presents how the results of concentration of hemoglobin, using the algorithm presented above, varies depending on the period of time passing between acquiring the blood sample into a cuvette and performing the absorption measurements. Fig. 3 shows the results for several different samples having different values of the concentration of hemoglobin. The thick line represents an average value of concentration of hemoglobin for all samples. The value returned by the algorithm is reduced quite substantially during the first seconds. This is due to drifting of the value of the absorption measurements. Thus, the absorption measurements are delayed.

The delay is achieved by allowing absorption measurements to be performed when it is confirmed that the drifting of the value of the absorption measurements has stopped. This may be done by monitoring the value of at least one of the absorption measurements for a period of time and, when the drifting has stopped, determining results of the absorption measurements to be used in processing.

Referring now to Fig. 4, a system implementing the above-described method will be described. The system comprises means 10 for emitting light at a first wavelength in a first range of 490 - 520 nm and at a second wavelength in a second range of 650 - 1200 nm. This means 10 for emitting light may be implemented by a combination of a light source emitting at several wavelengths or in broad wavelength ranges together with filters. Thus, the light source is arranged to emit light both at the first wavelength and at the second wavelength. Using the filter the wavelength emitted could selectively be controlled to be within one of these ranges. Alternatively, a first and a second light source may be used for emitting the first and the second wavelengths, respectively. Light emitting diodes may be used as light sources. Then, by switching the two light sources on and off, the means 10 for emitting light may be selectively controlled to emit light in the first or in the second wavelength.

Preferably, the first wavelength emitted by the means 10 for emitting light is in the range 500 - 510 nm, more preferably at 506 nm. Further, the second wavelength emitted by the means 10 for emitting light is preferably in the range 850 - 910 nm, and more preferably in the range 860 - 900 nm.

The system further comprises a cuvette holder 12 arranged to receive a capillary cuvette, which has an optical path length of less than 1 mm and holds a sample of unaltered whole blood. When a cuvette is placed in the holder 12, the optical window will be correctly positioned so that it will be irradiated with the light from the light source. Preferably, the cuvette holder 12 is arranged to receive a cuvette, which has an optical path length of less than 0.2 mm, and more preferably in the range 0.05 - 0.2 mm.

The system also comprises a controller for creating a delay of a determined period of time between placement of the cuvette in the cuvette holder and performing absorption measurements. The controller will thus ensure that a sufficient period of time passes from the acquiring of a sample into the cuvette and the performing of absorption measurements of the sample.

The controller comprises an analyser that monitors results of absorption measurements. The analyser may receive input from a detector 14 that detects light transmitted through the sample. When the analyser identifies that the result from the detector 14 becomes substantially constant, the analyser may conclude that the required period of time has passed. Thus, the results of the absorption measurements will now be stable and the controller may enable the control unit 13b such that the absorption measurements giving results to be processed may be initiated.

The light transmitted through the sample will be detected by a detector 14 so that a first absorption measurement may be obtained for light in the first range and a second absorption measurement may be obtained for light in the second range.

The system further comprises a processing unit 16 for processing results of the first and second absorption measurements to determine the concentration of hemoglobin in the sample according to the algorithm described above.

The system may suitably be implemented in a photometer comprising the means 10 for emitting light, the cuvette holder 12, and the detector 14. Photometers suitable for performing these measurements may be obtained by using photometers modified with suitable wave length filters and light emitting diodes. According to a preferred embodiment of the invention a photometer measures the absorbance at the two wavelengths and a built-in micro processor calculates, according to a programmed algorithm, the total concentration of hemoglobin in blood. Thus, no special absorption or interference filter which provide correction for variations in the detector sensitivity and in the effective optical path length as disclosed in WO 01/53806 are necessary.

In the above case, the processing unit 16 is embedded in the photometer. However, the processing unit 16 may also be connected to the photometer, and thus be implemented outside the photometer. For example, a computer connected to the photometer may be used.

The detector 14 may be arranged to detect essentially only directly transmitted light, since the scattered light need not be detected. This implies that the detector 14 detects light which is essentially within the diameter of the light beam irradiated on the sample and directly transmitted through the sample. Of course, some light may be scattered, while still being within this diameter. According to a preferred embodiment, the diameter of a detecting area of the detector 14 may typically be approximately 2 mm. The detector 14 is preferably arranged closer than 10 mm to the sample holder. This implies that light which has been scattered to small angles is detected.

**Claims**

1. A method for quantitative hemoglobin determination in undiluted, unhemolyzed whole blood comprising:

   acquiring a sample of unaltered whole blood into a capillary cuvette,
   presenting said cuvette to a set-up for an absorption measurement, wherein said presenting comprises placing the cuvette in a holder of an instrument for performing an absorption measurement,
   performing a first absorption measurement at a first wavelength in the range 490-520 nm directly on the sample in the cuvette,
   conducting a second absorption measurement at a second wavelength in the range 650 - 1200 nm and at which the absorption is substantially smaller than at the first wavelength, and
   processing results of the first and second absorption measurements to determine the concentration of hemoglobin in the sample, wherein said processing is performed by a predetermined algorithm,
   **characterized in that** the absorption measurement is delayed for a period of time, wherein said delaying is made by monitoring the value of at least one of the absorption measurements for a period of time and when the measurement results are substantially constant, allowing the first and second absorption measurements to be performed for determining the concentration of hemoglobin in the sample.

2. The method according to claim 1, wherein said processing determines the concentration of hemoglobin in the sample by computing the following formula:

$$[\text{Tot Hb}] = (Abs_1 - Abs_2) \cdot k_1 + k_2$$

wherein [Tot Hb] is the total concentration of hemoglobin in the sample, $Abs_1$ is the measured absorbance of the first absorption measurement, $Abs_2$ is the measured absorbance of the second absorption measurement, and $k_1$ and $k_2$ are calibration coefficients, which depend on the measurement arrangement.

3. The method according to any one of the preceding claims, wherein the first absorption measurement is performed at a wavelength in the range 500 - 510 nm, more preferably at 506 nm.

4. The method according to any one of the preceding claims, wherein the second absorption measurement is performed at a wavelength in the range 850 - 910 nm, more preferably in the range 860 - 900 nm.

5. The method according to any one of the preceding claims, wherein said cuvette has an optical path length of less than 1 mm, more preferably less than 0.2 mm.

6. The method according to claim 5, wherein said cuvette has an optical path length in the range 0.05 - 0.2 mm.

7. A system for quantitative hemoglobin determination in undiluted, unhemolyzed whole blood comprising:

means for emitting light (10) at a first wavelength in a first range of 490 - 520 nm and at a second wavelength in a second range of 650 - 1200 nm at which absorption of light in blood is substantially smaller than at the first wavelength,
a cuvette holder (12) arranged to receive a capillary cuvette, which holds a sample of unaltered whole blood,
a detector (14) for detecting light transmitted through the sample in a first absorption measurement for light in said first range and in a second absorption measurement for light in said second range,
a processing unit (16) for processing results of the first and second absorption measurements to determine the concentration of hemoglobin in the sample,
**characterized in that** the system comprises
a controller comprising an analyzer that monitors results of absorption measurements, and **in that**
the controller enables performing the first and second absorption measurements in response to the analyser identifying that the monitored results are substantially constant.

8. The system according to claim 7, wherein a detecting area of the detector (14) has a size such that essentially only directly transmitted light is detected.

9. The system according to any one of claims 7-8,
wherein the detector (14) is arranged closer than 10 mm to the cuvette holder (12).

**Patentansprüche**

1. Verfahren für die quantitative Hämoglobinbestimmung in unverdünntem, nicht hämolysiertem Vollblut, umfassend:

Abgeben einer Probe von unverändertem Vollblut in eine Kapillarküvette,
Präsentieren der Küvette einer Vorrichtung für eine Absorptionsmessung, wobei das Präsentieren das Platzieren der Küvette in einer Halterung eines Instruments zum Durchführen einer Absorptionsmessung umfasst,
Durchführen einer ersten Absorptionsmessung bei einer ersten Wellenlänge im Bereich von 490-520 nm direkt mit der Probe in der Küvette,

Durchführen einer zweiten Absorptionsmessung bei einer zweiten Wellenlänge im Bereich von 650-1200 nm, bei der die Absorption im Wesentlichen geringer ist als bei der ersten Wellenlänge, und

Verarbeitung der Ergebnisse der ersten und zweiten Absorptionsmessung zur Bestimmung der Hämoglobinkonzentration in der Probe, wobei die Verarbeitung von einem im Voraus festgelegten Algorithmus durchgeführt wird,

**dadurch gekennzeichnet, dass** die Absorptionsmessung eine Zeit lang verzögert wird, wobei das Verzögern erfolgt, indem der Wert mindestens einer der Absorptionsmessungen eine Zeit lang beobachtet wird, und die erste und die zweite Absorptionsmessung durchgeführt werden können, um die Hämoglobinkonzentration in der Probe zu bestimmen, wenn die Messergebnisse im Wesentlichen konstant sind.

2. Verfahren nach Anspruch 1, wobei die Verarbeitung die Hämoglobinkonzentration in der Probe bestimmt, indem die folgende Formel berechnet wird:

$$[\text{Gesamt-Hb}] = (Abs_1 - Abs_2) \cdot k_1 + k_2$$

wobei [Gesamt-Hb] die Gesamthämoglobinkonzentration in der Probe ist, $Abs_1$ die gemessene Absorption der ersten Absorptionsmessung ist, $Abs_2$ die gemessene Absorption der zweiten Absorptionsmessung ist und $k_1$ und $k_2$ Kalibrierungskoeffizienten sind, die von der Messanordnung abhängen.

3. Verfahren nach einem der vorhergehenden Ansprüche, wobei die erste Absorptionsmessung bei einer Wellenlänge im Bereich von 500-501 nm, insbesondere bei 506 nm durchgeführt wird.

4. Verfahren nach einem der vorhergehenden Ansprüche, wobei die zweite Absorptionsmessung bei einer Wellenlänge im Bereich von 850-910 nm, insbesondere im Bereich von 860-900 nm, durchgeführt wird.

5. Verfahren nach einem der vorhergehenden Ansprüche, wobei die Länge des optischen Pfads der Küvette weniger als 1 mm, insbesondere weniger als 0,2 mm, beträgt.

6. Verfahren nach Anspruch 5, wobei die Länge des optischen Pfads der Küvette im Bereich von 0,05-0,2 mm liegt.

7. System für die quantitative Hämoglobinbestimmung in unverdünntem, nicht hämolysiertem Vollblut, umfassend:

Mittel zum Abgeben von Licht (10) einer ersten Wellenlänge in einem ersten Bereich von 490-520 nm und einer zweiten Wellenlänge in einem zweiten Bereich von 650-1200 nm, bei der die Absorption von Licht im Blut im Wesentlichen geringer ist als bei der ersten Wellenlänge,

eine Küvettenhalterung (12), die angeordnet ist, um eine Kapillarküvette aufzunehmen, die eine Probe von unverändertem Vollblut enthält,

einen Detektor (14) zum Detektieren von Licht, das durch die Probe übertragen wird, in einer ersten Absorptionsmessung von Licht in dem ersten Bereich und in einer zweiten Absorptionsmessung von Licht in dem zweiten Bereich,

eine Verarbeitungseinheit (16) zum Verarbeiten von Ergebnissen der ersten und zweiten Absorptionsmessung, um die Hämoglobinkonzentration in der Probe zu bestimmen,

**dadurch gekennzeichnet, dass** das System eine Steuerungseinheit umfasst, welche einen Analysator umfasst, welcher Ergebnisse von Absorptionsmessungen überwacht, und dass

die Steuerungseinheit das Durchführen der ersten und zweiten Absorptionsmessung als Reaktion darauf, dass der Analysator feststellt, dass die überwachten Ergebnisse im Wesentlichen konstant sind, ermöglicht.

8. System nach Anspruch 7, wobei ein Detektionsbereich des Detektors (14) eine derartige Größe hat, dass im Wesentlichen nur direkt übertragenes Licht detektiert wird.

9. System nach einem der Ansprüche 7-8, wobei der Detektor (14) näher als 10 mm an der Küvettenhalterung (12) angeordnet ist.

**Revendications**

1. Procédé de détermination quantitative de l'hémoglobine dans du sang total non dilué, non hémolysé, qui comprend :

   l'acquisition d'un échantillon de sang total non modifié dans une cuvette capillaire,
   la présentation de ladite cuvette à une installation pour une mesure d'absorption, dans lequel ladite présentation comprend le fait de placer la cuvette dans un support d'un instrument pour réaliser une mesure d'absorption,
   la réalisation d'une première mesure d'absorption à une première longueur d'onde dans la plage de 490 à 520 nm directement sur l'échantillon dans la cuvette,
   le fait de réaliser une seconde mesure d'absorption à une seconde longueur d'onde dans la plage de 650 à 1200 nm et à laquelle l'absorption est substantiellement inférieure à celle à la première longueur d'onde, et
   le traitement des résultats des première et seconde mesures d'absorption pour déterminer la concentration de l'hémoglobine dans l'échantillon, dans lequel ledit traitement est réalisé par un algorithme prédéterminé, **caractérisé en ce que** la mesure d'absorption est retardée pendant une période de temps, dans lequel ledit délai est réalisé en surveillant la valeur d'au moins une des mesures d'absorption pendant une certaine durée et lorsque les résultats des mesures sont substantiellement constants, en permettant la réalisation des première et seconde mesures d'absorption pour déterminer la concentration de l'hémoglobine dans l'échantillon.

2. Procédé selon la revendication 1, dans lequel ledit traitement détermine la concentration de l'hémoglobine dans l'échantillon par calcul de la formule suivante :

$$[\text{Tot Hb}] = (Abs_1 - Abs_2) \cdot k_1 + k_2$$

   dans laquelle [Tot Hb] est la concentration totale de l'hémoglobine dans l'échantillon, $Abs_1$ est l'absorbance mesurée de la première mesure d'absorption, $Abs_2$ est l'absorbance mesurée de la seconde mesure d'absorption, et $k_1$ et $k_2$ sont des coefficients d'étalonnage, qui dépendent du dispositif de mesure.

3. Procédé selon l'une quelconque des revendications précédentes, dans lequel la première mesure d'absorption est réalisée à une longueur d'onde dans la plage de 500 à 510 nm, de préférence à 506 nm.

4. Procédé selon l'une quelconque des revendications précédentes, dans lequel la seconde mesure d'absorption est réalisée à une longueur d'onde dans la plage de 850 à 910 nm, de préférence dans la plage de 860 à 900 nm.

5. Procédé selon l'une quelconque des revendications précédentes, dans lequel ladite cuvette a une longueur de trajectoire optique inférieure à 1 mm, de préférence inférieure à 0,2 mm.

6. Procédé selon la revendication 5, dans lequel ladite cuvette a une longueur de trajectoire optique dans la plage de 0,05 à 0,2 mm.

7. Système de détermination quantitative de l'hémoglobine dans du sang total non dilué, non hémolysé, qui comprend :

   un moyen d'émission de lumière (10) à une première longueur d'onde dans la plage de 490 à 520 nm et à une seconde longueur d'onde dans une seconde plage de 650 à 1200 nm à laquelle l'absorption de la lumière dans le sang est substantiellement inférieure à celle à la première longueur d'onde,
   un support de cuvette (12) configuré pour recevoir une cuvette capillaire, qui contient un échantillon de sang total non modifié,
   un détecteur (14) pour détecter la lumière transmise à travers l'échantillon dans une première mesure d'absorption pour la lumière dans ladite première plage et dans une seconde mesure d'absorption pour la lumière dans ladite seconde plage,
   une unité de traitement (16) pour le traitement des résultats des première et seconde mesures d'absorption pour déterminer la concentration de l'hémoglobine dans l'échantillon, **caractérisé en ce que** le système comprend :

      un dispositif de commande comprenant un analyseur qui surveille les résultats de mesures d'absorption, et **en ce que**

le dispositif de commande permet de réaliser les première et seconde mesures d'absorption en réponse à l'identification par l'analyseur du fait que les résultats surveillés sont substantiellement constants.

8. Système selon la revendication 7, dans lequel une zone de détection du détecteur (14) a une taille telle que sensiblement uniquement la lumière directement transmise est détectée.

9. Système selon l'une quelconque des revendications 7 à 8, dans lequel le détecteur (14) est disposé à moins de 10 mm du support de cuvette (12).

Fig. 1

*Fig. 2*

*Fig. 4*

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4088448 A
- US 5674457 A
- US 6638769 B
- US 5064282 A
- US 6262798 B
- WO 0153806 A
- US 6262798 A
- US 5898487 A
- US 6084661 A
- US 2005036147 A
- US 6831733 B

**Non-patent literature cited in the description**

- **VANZETTI, G.** An azide-methaemoglobin method for haemoglobin determination in blood. *Am.J. Lab.& Clin. Med.,* 1966, vol. 67, 116-126
- **VON SCHENCK, H. ; FALKENSSON, M. ; LUND-BERG, B.** Evaluation of 'HemoCue', a new device for determining hemoglobin. *Clinical Chemistry,* 1986, vol. 32 (3), 526-529